# EUROPÄISCHE PATENTANMELDUNG

(11) **EP 1 110 573 A2**
(43) Veröffentlichungstag der Anmeldung: **27.06.2001**
(21) Anmeldenummer: 00127043.8
(22) Anmeldetag: 09.12.2000
(51) Int. Cl.: A61M 16/08

(54) **Vorrichtung zur Zufuhr eines Atemgases**

(30) Priorität: 23.12.1999 DE 19962516
(71) Anmelder: MAP Medizintechnik für Arzt und Patient GmbH & Co. KG, 82152 Martinsried (DE)
(72) Erfinder: Genger, Harald, 82319 Starnberg (DE); Mayer, Wolfgang, 79285 Ebringen (DE)
(74) Vertreter: Rössig, Rolf

(57) **Zusammenfassung**

Die Erfindung betrifft eine Vorrichtung zur Zufuhr eines Atemgases unter Überdruck zu einem Patienten, wie sie beispielsweise zur Behandlung schlafbezogener Atmungsstörungen im Rahmen einer CPAP-Therapie Anwendung finden kann. Die erfindungsgemäße Vorrichtung umfaßt eine Atemgaswegeinrichtung, die einen Atemgasansaugabschnitt und einen Atemgasabgabeabschnitt sowie eine Fördereinrichtung zur Förderung des Atemgases aus dem Atemgasansaugabschnitt zu dem Atemgasabgabeabschnitt sowie eine Gehäuseeinrichtung zur Aufnahme der Fördereinrichtung aufweist. Erfindungsgemäß ist eine Kammereinrichtung vorgesehen, die mit der Atemgasleitungseinrichtung, insbesondere dem Atemgasansaugabschnitt akustisch gekoppelt ist. Hierdurch wird auf vorteilhafte Weise eine Ausbreitung der Laufgeräusche der Fördereinrichtung nach außen vermieden, ohne daß hierbei der respiratorische Widerstand des CPAP-Gerätes zunimmt.

## Beschreibung

Die Erfindung betrifft eine Vorrichtung zur Zufuhr eines Atemgases zu einem Patienten, wie sie beispielsweise im Rahmen einer CPAP-Therapie zur Behandlung schlafbezogener Atmungsstörungen Anwendung finden kann.

Zur Durchführung einer CPAP-Therapie wird einem Patienten ein Atemgas - insbesondere Umgebungsluft - unter einem vorbestimmten Überdruck zugeführt.

Die Förderung des Atemgases, insbesondere der Umgebungsluft, erfolgt bei den heute üblichen CPAP-Geräten mittels einer drehzahlgesteuerten Gebläseeinrichtung, die das Atemgas über einen Atemgasansaugabschnitt ansaugt und an einen Atemgasabgabeabschnitt weiterleitet. Die bekannten CPAP-Geräte sind üblicherweise über eine flexible Schlauchleitung mit einer Atemmaske verbunden, welche wiederum in abdichtender Weise auf den Nasen-und/oder Mundbereich des Patienten aufsetzbar ist.

Durch die Zufuhr des Atemgases unter einem vorbestimmten Überdruck wird auf vorteilhafte Weise eine pneumatische Schienung der oberen Atemwege erreicht, wodurch auf nachweisbar zuverlässige Weise etwaigen Atemwegsobstruktionen in diesem Bereich vorgebeugt werden kann.

Die Behandlung schlafbezogener Atmungsstörungen im Rahmen einer CPAP-Therapie läßt sich mit einer entsprechenden Geräteausstattung ambulant im häuslichen Bereich durchführen. Der Austausch der ausgeatmeten CO₂-Atemgasfraktion erfolgt üblicherweise über ein kallibriertes Auslaßorgan, das entweder in eine Atemmaske integriert ist, oder zumindest in unmittelbarer Nähe des Patienten angeordnet ist. Der über dieses Auslaßorgan abströmende Gasstrom sowie das CPAP Gerät selbst, führen allgemein zu Geräuschemissionen. Es hat sich gezeigt, daß insbesondere bei einem geringem Umgebungsgeräuschpegel diese Betriebsgeräusche nur bedingt toleriert werden. Im Hinblick darauf, daß im Rahmen einer Expirationsphase das Atemgas infolge des durch den Patienten aufgebrachten Expirationsdruck zum CPAP-Gerät zurück gefördert wird, erweist sich die CPAP-geräteseitige Schalldämmung als problematisch.

Unter dem Eindruck dieses Problems liegt der Erfindung die Aufgabe zugrunde, ein CPAP-Gerät zu schaffen, das sich bei weiterhin vergleichsweise geringem respiratorischem Widerstand durch eine geringe Schallemission auszeichnet.

Diese Aufgabe wird erfindungsgemäß gelöst durch eine Vorrichtung zur Zufuhr eines Atemgases unter Überdruck mit einer Atemgaswegeinrichtung die einen Atemgasansaugabschnitt und einen Atemgasabgabeabschnitt umfaßt, einer Fördereinrichtung zur Förderung des Atemgases aus dem, Atemgasansaugabschnitt zu dem Atemgasabgabeabschnitt und einer Gehäuseeinrichtung zur Aufnahme der Atemgasfördereinrichtung, die sich dadurch auszeichnet, daß eine Kammereinrichtung vorgesehen ist, die mit der Atemgasleitungseinrichtung akustisch gekoppelt ist.

Dadurch wird es auf vorteilhafte Weise möglich, die Ausbreitung der seitens der Gebläseeinrichtung hervorgerufenen Schallereignisse über die Atemgasleitungseinrichtung deutlich zu unterdrücken, ohne daß hierbei der Strömungswiderstand der Atemgaswegeinrichtung und damit auch nicht der respiratorische Widerstand des CPAP-Gerätes zunimmt.

Gemäß einer besonders bevorzugten Ausführungsform der Erfindung ist die Kammereinrichtung mit dem Atemgasansaugbereich akustisch gekoppelt. Dadurch wird es auf vorteilhafte Weise möglich, die Ausbreitung der Gebläsegeräusche über den Atemgasansaugabschnitt zu verhindern.

Die akustische Koppelung der Kammereinrichtung mit der Atemgaswegeinrichtung erfolgt gem. einer besonders bevorzugten Ausführungsform der Erfindung dadurch, daß die Kammereinrichtung unmittelbar in die Atemgaswegeinrichtung integriert ist. In vorteilhafter Weise sind hierzu eine Zuströmöffnung und eine Abströmöffnung vorgesehen, wobei diese beiden Öffnungen vorzugsweise bezogen auf die allgemeinen Dimensionen der Kammereinrichtung vergleichsweise nahe - vorzugsweise einander unmittelbar benachbart - angeordnet sind.

Die Raumakustik der Kammereinrichtung ist gem. einer besonders bevorzugten Ausführungsform der Erfindung derart abgestimmt, daß insbesondere für das hörbare Frequenzspektrum der Gebläsegeräusche eine weitgehende Schallextinktion erreicht wird. Der Schallextinktionserfolg wird neben der Dimensionierung der Kammereinrichtung auch durch den Ort der Ankoppelung der Atemgaswegeinrichtung an die Kammereinrichtung bestimmt.

Besonders gute Schallextinktionserfolge werden bei einer besonders bevorzugten Ausführungsform der Erfindung dadurch erreicht, daß die Kammereinrichtung als im wesentlichen quaderförmige Kammer ausgebildet ist. Die Seitenwandungen der Kammer können zumindest abschnittsweise mit einer Oberflächenstruktur versehen sein, die eine vorbestimmte Schallreflexions-, bzw. Schallabsorptionscharakteristik aufweist.

Eine besonders effektive Schallextinktion kann insbesondere bei dieser quaderförmigen Kammer dadurch erreicht werden, daß die Ankoppelung der Kammereinrichtung an den Atemgasweg in einem Eckbereich der Kammereinrichtung erfolgt. Diese Ausführungsform erweist sich insbesondere bei einer Ankoppelung der Kammereinrichtung an den Atemgasansaugabschnitt als besonders wirkungsvoll.

Die erfindungsgemäß vorgeschlagene akustisch aktive Kammereinrichtung läßt sich gem. einer besonders bevorzugten Ausführungsform im Bodenbereich der Gehäuseeinrichtung des CPAP-Gerätes anordnen. Hierzu ist es möglich, eine obere Begrenzungsfläche der Kammereinrichtung unmittelbar durch einen Bodenabschnitt der Gehäuseeinrichtung auszubilden, wobei der untere Bereich der Kammereinrichtung durch ein Deckelelement begrenzt ist. Dieses Deckelelement kann beispielsweise von außen in abdichtender Weise auf den Bodenbereich des CPAP-Gerätes aufgesetzt, insbesondere aufgeschraubt werden.

Die akustischen Eigenschaften der Kammereinrichtung sind gem. einer besonders bevorzugten Ausführungsform der Erfindung durch Abstimmung der jeweiligen Schall-Lauflängen derart abgestimmt, daß im Ansaugabschnitt des CPAP-Gerätes - zumindest im hörbaren Frequenzbereich der Gebläseeinrichtung eine weitgehende Schallextinktion erfolgt. Eine besonders gezielte Beeinflussung der akustischen Eigenschaften der Kammereinrichtung kann gem. einer besonders bevorzugten Ausführungsform der Erfindung dadurch erreicht werden, daß ausgewählte Innenwandungsabschnitte der Kammereinrichtung mit einem schallabsorbierenden Material verkleidet sind. Es ist auch möglich, insbesondere bei der Herstellung der Gehäuseeinrichtung aus einem Kunststoffmaterial die Schallreflexionseigenschaften der Kammereinrichtung sowie die Schallabsorptionseigenschaften bestimmter Wandungsabschnitte durch ausgewählte Oberflächenrauhigkeiten sowie ausgewählte Mikrostrukturen der Wandungsabschnitte definiert festzulegen.

Die Kammereinrichtung ist gem. einer besonders bevorzugten Ausführungsform derart dimensioniert, daß die Reflexionscharakteristik der Kammerwandung zu einer Schalldruckverteilung führt, die eine akustische Abkoppelung des von außen herbeigeführten Eintrittsbereiches des Ansaugabschnittes von dem gebläsenahen Bereich des Ansaugabschnittes bewirkt. In vorteilhafter Weise weist hierzu die Kammereinrichtung eine Trennwandanordnung auf, die innerhalb der Kammereinrichtung Schallwegabschnitte definiert, deren Schall-Laufzeit einen Versatz von ca. λ/2 aufweist, wobei λ der Schallwellenlänge von Frequenzanteilen hoher Schallintensität entspricht.

Die Länge der Kammereinrichtung liegt vorzugsweise im Bereich von ca. 120 - 170 mm. Die Breite der Kammereinrichtung liegt vorzugsweise im Bereich von 70 - 120 mm, und die Tiefe der Kammereinrichtung liegt vorzugsweise im Bereich von 7 - 30 mm. Es ist auch möglich, bei der Gestaltung der Kammereinrichtung von einer im wesentlichen quaderförmigen Grundstruktur abzuweichen und das zu einer weitgehenden Schallabkoppelung führende Schallereignis im Inneren der Kammer durch anders angeordnete Kammerwandungsabschnitte zu erreichen.

Weitere vorteilhafte Ausgestaltungen der Erfindung sind Gegenstand der Unteransprüche.

Weitere Einzelheiten der Erfindung ergeben sich aus der nachfolgenden Beschreibung eines bevorzugten Ausführungsbeispiels in Verbindung mit der Zeichnung.

Es zeigen:
- **Fig. 1**: eine perspektivische Ansicht des Bodenbereiches eines erfindungsgemäßen CPAP-Gerätes mit im Eckbereich vorgesehener Ankoppelungsstelle zur akustischen Ankoppelung eines Atemgasansaugabschnittes;
- **Fig. 2**: eine perspektivische Ansicht eines Deckelelements zum Aufsatz auf den Bodenbereich des CPAP-Gerätes gem. Fig. 1.

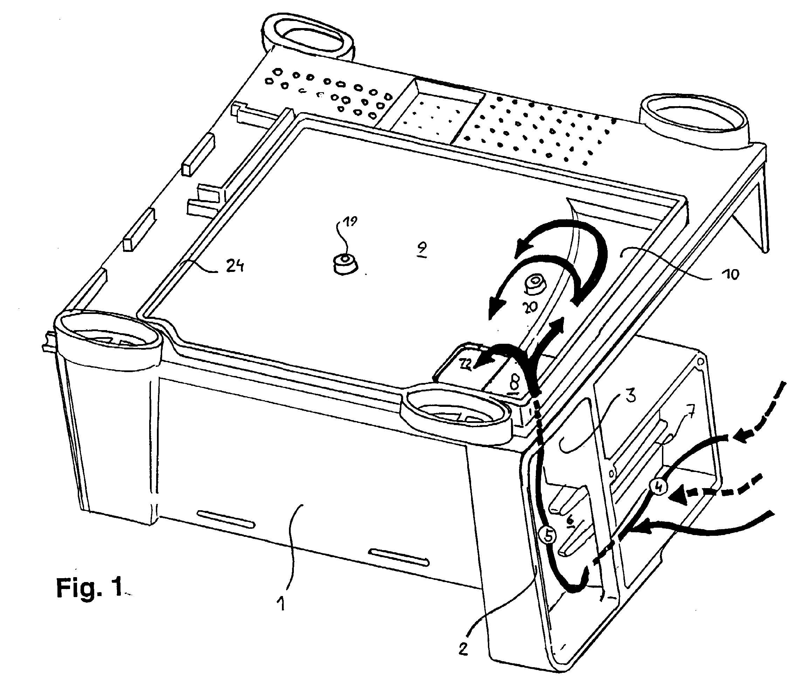

In Fig. 1 ist eine perspektivische Ansicht des Bodenabschnittes einer Gehäuseeinrichtung eines CPAP-Gerätes dargestellt. In dieses dreidimensionale Strukturbauteil 1 sind durch integrale Wandungsabschnitte 2, 3 Atemgaswege eingeformt, über welche das Atemgas (hier Umgebungsluft) auf besonders geräuscharme Weise zu einer Gebläseeinrichtung (nicht dargestellt) gefördert werden kann. Diese Atemgaswege können mit einem schallabsorbierenden Material ausgekleidet werden.

Bei der hier dargestellten Ausführungsform strömt das angesaugte Atemgas über einen Ansaugabschnitteintrittsbereich 4, der hier im Seitenbereich des CPAP-Gerätes angeordnet ist, in einen zum Bodenbereich des CPAP-Gerätes abwärts führenden Ansaugwegabschnitt 5. Die Zuströmung der Umgebungsluft zu dem Ansaugwegabschnitt 5 erfolgt über eine vergleichsweise große Übergangsmündung 6. Im Bereich des Ansaugabschnitteintrittsbereichs 4 sind bei der hier dargestellten Ausführungsform Atemgasführungsvorsprünge 7 vorgesehen, durch welche die Zuströmung der Umgebungsluft in den Ansaugwegabschnitt 5 unterstützt wird.

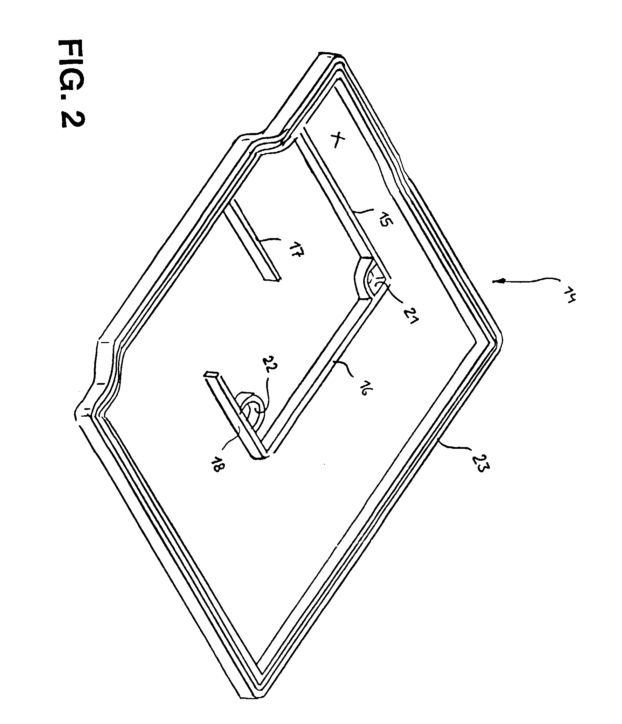

Die durch den Ansaugwegabschnitt 5 strömende Umgebungsluft gelangt über eine Kammermündung 8 in die akustisch aktive Schallextinktionskammer 9. Die Kammermündung 8 mündet hierbei in einen hier leicht versenkt ausgebildeten Kanalabschnitt 10, der über seine gesamte Länge mit der akustisch aktiven Schallextinktionskammer 9 in Verbindung steht. Die hier akustisch aktive Schallextinktionskammer 9 ist durch eine im wesentlichen rechteckquaderförmige Kammereinrichtung gebildet, deren Oberseite durch die Unterseite des Strukturbauteils 1 gebildet ist. Der Bodenbereich der Kammereinrichtung ist durch ein Deckelelement 14 (Fig. 2) gebildet.

In unmittelbarer Nachbarschaft zu der Kammermündung 8 ist eine Austrittsöffnung 12 vorgesehen, über welche die zumindest teilweise über den Kanalabschnitt 10 verteilte, angesaugte Luft aus der akustisch aktiven Kammereinrichtung 9 abgesaugt wird. Im Anschluß an diese Öffnung 12 ist ein weiterer zur Gebläseeinrichtung führender Leitungsabschnitt (nicht dargestellt) vorgesehen, der vorzugsweise mit einem schallabsorbierenden Material ausgekleidet ist.

Die bei der derart getroffenen Anordnung von der Gebläseeinrichtung erzeugten, zur Öffnung 12 entgegen der Atemgasströmungsrichtung sich ausbreitenden Schallereignisse gelangen über die Mündung 12 in die akustisch aktive Schallextinktionskammer 9. Im Inneren dieser Schallextinktionskammer 9 wird im wesentlichen aufgrund der Laufzeitunterschiede der Schallereignisse eine Überlagerung der Schallereignisse erreicht, die im Bereich der Kammermündung 8 zu einer weitgehenden Schallextinktion zumindest bestimmter Wellenlängen führt. Hierdurch wird auf vorteilhafte Weise vermieden, daß sich die unerwünschten Laufgeräusche und Strömungsgeräusche der Fördereinrichtung über den Atemgasansaugabschnitt 5, 4 nach außen hin ausbreiten.

Die akustischen Eigenschaften der Schallextinktionskammer 9 können durch die Dimensionierung der Kammereinrichtung sowie durch die willkürliche Anordnung von SchallreflektionsWandungen auf die spezielle Gerätekonfiguration abgestimmt werden.

In Fig. 2 ist hierzu ein Deckelelement 14 dargestellt, das zum einen die untere Begrenzung der Schallextinktionskammer 9 bildet und zum anderen zugleich entsprechende Reflexionswandungen 15, 16, 17 und 18 aufweist. Der bei diesem Deckelelement durch den Buchstaben x gekennzeichnete Deckelabschnitt befindet sich beim montierten Deckelelement 14 in unmittelbarer Nähe der Kammermündung 8. Durch die Schallreflektionswandungen 15, 16, 17 und 18 werden Laufwege definiert, deren Schall-Laufzeit derart abgestimmt ist, daß sich die Schallereignisse im Bereich x nahezu vollständig auslöschen.

Alternativ zu dem der hier dargestellten Ausgestaltung der Schallextinktionskammer 9 verwirklichten, auf Schallreflexionsereignissen basierenden Extinktionsprinzip ist es auch möglich, die Schallextinktionskammer mit einem schallabsorbierenden Material auszukleiden. Auch hierbei wird auf überraschend vorteilhafte Weise eine extrem effektive Schallauslöschung erreicht, da aufgrund der in der Kammer stark abgesenkten Strömungsgeschwindigkeit eine vergleichsweise lange Verweilzeit und damit effektive Schallauslöschung erreicht wird.

Bei der hier dargestellten Ausführungsform der Erfindung sind sowohl das Strukturbauteil 1 als auch das Deckelelement 14 aus einem Kunststoffmaterial gebildet. Das Strukturbauteil 1 und das Deckelelement 14 sind im wesentlichen zentral durch Verbindungseinrichtungen 19, 20, 21, 22 miteinander koppelbar. Durch die zentrale Anordnung der Verbindungseinrichtungen, insbesondere von Schrauben, wird auf vorteilhafte Weise vermieden, daß diese großen Begrenzungsflächen der Kammereinrichtung in Schwingung geraten.

Eine besonders hohe Laufruhe des CPAP-Gerätes kann weiterhin dadurch erreicht werden, daß das in Fig. 2 dargestellte Deckelelement 14 aus einem Material mit einem hohen spezifischen Gewicht, beispielsweise Metall, gebildet ist. Eine besonders passgenaue und schalldichte Koppelung des Deckelelementes 14 und des Strukturbauteils 1 kann dadurch erreicht werden, daß die Deckeleinrichtung 14 mit einer Randstruktur 23 versehen ist, die in enger Passung abdichtend auf eine entsprechende Gegenstruktur 24 des Strukturbauteils 1 aufsetzbar ist.

Die akustisch aktive Kammereinrichtung ist vorzugsweise derart an den Atemgasweg angekoppelt, daß der Atemgasströmungsweg nur über eine kurze Strecke durch die Kammer verläuft. Vorzugsweise befinden sich die hierzu vorgesehene Eintrittsmündung 8 und die Austrittsmündung 12 in unmittelbarer Nähe. Der übrige Bereich des in der Kammer eingeschlossenen Gasvolumens nimmt nur allmählich an dem die Kammer durchsetzenden Gasstrom teil.

Die Erfindung ist nicht auf das vorangehend beschriebene Ausführungsbeispiel beschränkt. Obgleich es sich im Hinblick auf eine besonders kompakte Ausgestaltung des CPAP-Gerätes als vorteilhaft erweist, die großvolumige Schallextinktionskammer im Bodenbereich des CPAP-Gerätes auszubilden, ist es auch möglich, diese Schallextinktionskammer im Inneren des CPAP-Gerätes, insbesondere im oberen Bereich des CPAP-Gerätes auszubilden. Es ist auch möglich, mehrere derartige Schallextinktionskammern sowie insbesondere eine derartige Schallextinktionskammer für den stromabwärts der Fördereinrichtung liegenden Atemgasweg vorzusehen.

## Patentansprüche

1. Vorrichtung zur Zufuhr eines Atemgases unter Überdruck mit :
- einer Atemgaswegeinrichtung die einen Atemgasansaugabschnitt, und einen Atemgasabgabeabschnitt umfaßt,
- einer Gebläseeinrichtung zur Förderung des Atemgases aus dem, Atemgasansaugabschnitt zu dem Atemgasabgabeabschnitt, und
- einer Gehäuseeinrichtung zur Aufnahme der Fördereinrichtung,
dadurch gekennzeichnet, daß eine Kammereinrichtung vorgesehen ist, die mit der Atemgasleitungseinrichtung akustisch gekoppelt ist.

2. Vorrichtung nach Anspruch 1, dadurch gekennzeichnet, daß die Kammereinrichtung eine akustisch aktive Schallextinktionskammer bildet, zur Auslöschung der sich gegen die Atemgasansaugrichtung ausbreitenden Laufgeräusche der Gebläseeinrichtung.

3. Vorrichtung nach Anspruch 1 oder 2, dadurch gekennzeichnet, daß die Kammereinrichtung mit dem Atemgasansaugbereich akustisch gekoppelt ist.

4. Vorrichtung nach wenigstens einem der Ansprüche 1 bis 3, dadurch gekennzeichnet, daß die Kammereinrichtung in die Atemgaswegeinrichtung integriert ist.

5. Vorrichtung nach wenigstens einem der Ansprüche 1 bis 4, dadurch gekennzeichnet, daß die Raumakustik der Kammereinrichtung und der Ort der Ankoppelung an die Atemgaswegeinrichtung derart festgelegt sind, daß zumindest für das hörbare Frequenzspektrum der Gebläsegeräusche eine weitgehende Schallextinktion erfolgt.

6. Vorrichtung nach wenigstens einem der Ansprüche 1 bis 5, dadurch gekennzeichnet, daß die Kammereinrichtung durch eine im wesentlichen quaderförmige Kammer gebildet ist.

7. Vorrichtung nach wenigstens einem der Ansprüche 1 bis 6, dadurch gekennzeichnet, daß die Kammereinrichtung in einem Eckbereich an den Atemgasweg angekoppelt ist.

8. Vorrichtung nach wenigstens einem der Ansprüche 1 bis 7, dadurch gekennzeichnet, daß die Kammereinrichtung an den Atemgasansaugabschnitt angekoppelt ist.

9. Vorrichtung nach wenigstens einem der Ansprüche 1 bis 8, dadurch gekennzeichnet, daß die Innenwandung der Kammereinrichtung eine vorbestimmte Schallreflektionscharakteristik aufweist.

10. Vorrichtung nach wenigstens einem der Ansprüche 1 bis 9, dadurch gekennzeichnet, daß die Kammereinrichtung im Bodenbereich der Gehäuseeinrichtung vorgesehen ist.

11. Vorrichtung nach wenigstens einem der Ansprüche 1 bis 10, dadurch gekennzeichnet, daß die Kammereinrichtung zwischen dem Bodenbereich und einem Deckelelement begrenzt ist.

12. Vorrichtung nach wenigstens einem der Ansprüche 1 bis 11, dadurch gekennzeichnet, daß das Deckelelement von außen auf den Bodenbereich aufgesetzt ist.

13. Vorrichtung nach wenigstens einem der Ansprüche 1 bis 12, dadurch gekennzeichnet, daß die Kammereinrichtung ein akustisches Ereignis hervorruft das im Ansaugabschnitt zu einer weitgehenden Schallextinktion im hörbaren Frequenzbereich führt.

14. Vorrichtung nach wenigstens einem der Ansprüche 1 bis 13, dadurch gekennzeichnet, daß ausgewählte Innenwandungsabschnitte der Kammereinrichtung mit einem schallabsorbierenden Material verkleidet sind.

15. Vorrichtung nach wenigstens einem der Ansprüche 1 bis 14, dadurch gekennzeichnet, daß die Kammereinrichtung derart dimensioniert ist, daß die Reflektionscharakteristik der Kammerwandung zu einer Schalldruckverteilung führt die eine akustische Abkoppelung des Eintrittsbereiches des Ansaugabschnittes von der dem gebläsenahen Bereich des Ansaugabschnittes bewirkt.

16. Vorrichtung nach wenigstens einem der Ansprüche 1 bis 15, dadurch gekennzeichnet, daß die Kammereinrichtung mit einer Trennwandanordnung versehen ist, die innerhalb der Kammereinrichtung Schallwegabschnitte definiert deren Schall-Laufzeitdifferenz im Bereich von λ/2 liegt.

17. Vorrichtung nach wenigstens einem der Ansprüche 1 bis 16, dadurch gekennzeichnet, daß die akustische Aktivität der Kammereinrichtung zu einer Schallextinktion im Bereich des Atemgasansaugabschnittes führt.

18. Vorrichtung nach wenigstens einem der Ansprüche 1 bis 17, dadurch gekennzeichnet, daß die Länge der Kammereinrichtung im Bereich von 120 bis 170mm liegt, und daß die Breite der Kammereinrichtung im Bereich von 70 bis 140mm liegt, und daß die Tiefe der Kammer im Bereich von 7 bis 30mm liegt.

19. Vorrichtung nach wenigstens einem der Ansprüche 1 bis 18, dadurch gekennzeichnet, daß der Ansaugluftweg eine erste zur Geräterückseite hin offene Rinne (4) und einen in Strömungsrichtung abfolgenden, zum Bodenbereich des Gerätes hinabführenden, Kanal (5) aufweist, der in die akustisch aktive Kammereinrichtung (9) führt.
